# EUROPÄISCHE PATENTSCHRIFT

(11) **EP 2 049 253 B1**
(45) Veröffentlichungstag und Bekanntmachung des Hinweises auf die Patenterteilung: **11.09.2019**
(21) Anmeldenummer: 07787686.0
(22) Anmeldetag: 18.07.2007
(51) Int. Cl.: B01J 23/46, B01J 38/04, C07C 5/10, C07C 51/36, C07C 67/303

(54) **integriertes VERFAHREN bestehend aus Hydrierung mit RUTHENIUMKATALYSATOREN und REGENERIERUNG derselben**
integrated process comprising dehydration using ruthenium catalysts and regneration thereof
procede integre comprenant la dehydratation utilisant un catalyseur de ruthenium et regeneration correspondante

(30) Priorität: 31.07.2006 EP 06118206
(43) Veröffentlichungstag der Anmeldung: 22.04.2009
(73) Patentinhaber: BASF SE, 67056 Ludwigshafen am Rhein (DE)
(72) Erfinder: HENKELMANN, Jochem, 68165 Mannheim (DE); BECKER, Michael, 77654 Offenburg (DE); MIRK, Daniela, 68161 Mannheim (DE); RICHTER, Felix, 67069 Ludwigshafen (DE); SCHÄFER, Thomas, 68165 Mannheim (DE)
(74) Vertreter: BASF IP Association
(86) Internationale Anmeldenummer: PCT/EP2007/057428
(87) Internationale Veröffentlichungsnummer: WO 2008/015103

(56) Entgegenhaltungen:
- EP-A- 0 913 194
- EP-A1- 0 696 574
- WO-A-03/010119
- DE-A1- 10 128 242
- DE-A1- 10 216 745
- JP-A- 1 159 059
- JP-A- 2000 051 701
- DATABASE WPI Section Ch, Week 199118 Derwent Publications Ltd., London, GB; Class E19, AN 1991-129075 XP002456155 & JP 03 068453 A (ASAHI CHEM IND CO LTD) 25. März 1991 (1991-03-25)

## Beschreibung

Die vorliegende Erfindung betrifft ein integriertes Verfahren zur Hydrierung und Regenerierung eines Rutheniumkatalysators, der insbesondere zur Hydrierung von gegebenenfalls substituierten ein- oder mehrkernigen Aromaten zu den entsprechenden Cycloaliphaten geeignet ist.

Es existieren zahlreiche Verfahren zur Hydrierung von Aromaten, etwa von Benzol zu Cyclohexan. Diese Hydrierungen werden überwiegend an Nickel- und Platinkatalysatoren in der Gas- oder Flüssigphase durchgeführt. Derartige Hydrierverfahren sind unter anderem in US 3,597,489, US 2,898,387 beziehungsweise GB 799 396 offenbart. Typischerweise wird dabei zunächst in einem Hauptreaktor der größte Teil des Benzols zu Cyclohexan hydriert und anschließend in einem oder mehreren Nachreaktoren die Umsetzung zu Cyclohexan komplettiert.

Ein besonders geeigneter Katalysator, der bei der Hydrierung aromatischer Verbindungen eingesetzt werden kann, wird in der DE 196 24 485 A1 offenbart. Der Katalysator umfasst als Aktivmetall Ruthenium alleine oder zusammen mit mindestens einem Metall der I., VII., oder VIII. Nebengruppe des Periodensystems (CAS-Version) in einer Menge von 0,01 bis 30 Gew.-% bezogen auf das Gesamtgewicht des Katalysators und ist auf einen Träger aufgebracht. 10 bis 50% des Porenvolumens des Trägers werden von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet, wobei sich die Summe der Porenvolumina zu 100% addiert. Als Träger werden Aktivkohle, Siliziumcarbid, Aluminiumoxid, Siliziumdioxid, Titandioxid, Zirkoniumdioxid, Magnesiumdioxid, Zinkoxid oder ein Gemisch aus zwei oder mehr davon eingesetzt.

Weitere besonders geeignete Katalysatoren zur Hydrierung aromatischer Verbindungen werden in der EP-A 1 169 285 offenbart. Der Katalysator umfasst in einer Ausführungsform (Katalysator 1) mindestens ein Metall der VIII. Nebengruppe des Periodensystems (CAS-Version), aufgebracht auf einen Träger, wobei der Träger Makroporen aufweist und der Katalysator als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfasst, wobei der Träger einen mittleren Porendurchmesser von mindestens 50 nm und eine BET-Oberfläche von höchstens 30 m²/g aufweist und die Menge des Aktivmetalls 0,01 bis 30 Gew.- %, bezogen auf das Gesamtgewicht des Katalysator, beträgt. In einer weiteren Ausführungsform (Katalysator 2) umfasst der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei die Summe der Anteile der Porenvolumina zu 100% beträgt. Als Träger werden Aktivkohle, Siliziumcarbid, Aluminiumoxid, Siliziumdioxid, Titandioxid, Zirkoniumdioxid, Magnesiumdioxid, Zinkoxid oder ein Gemisch aus zwei oder mehr davon eingesetzt, vorzugsweise Aluminiumoxid.

Schließlich ist ein weiterer besonders geeigneter Katalysator in der Anmeldung DE 102 005 029 200 offenbart. Es handelt sich um einen Schalenkatalysator enthaltend als Aktivmetall Ruthenium alleine oder zusammen mit mindestens einem weiteren Metall der Nebengruppen IB, VIIB oder VIII des Periodensystems der Elemente (CAS-Version), aufgebracht auf einen Träger enthaltend Siliziumdioxid als Trägermaterial, dadurch gekennzeichnet, dass die Menge des Aktivmetalls < 1 Gew.-% beträgt, bezogen auf das Gesamtgewicht des Katalysators, und mindestens 60 Gew.-% des Aktivmetalls in der Schale des Katalysators bis zu einer Eindringtiefe von 200 µm vorliegen, ermittelt mittels SEM-EPMA (EDXS).

Der Erhalt der Katalysatoraktivität über einen möglichst langen Zeitraum ist für industrielle Prozesse von großer wirtschaftlicher Bedeutung.

Üblicherweise wird ein Nachlassen der katalytischen Aktivität durch verschiedene physikalische und chemische Effekte auf den Katalysator hervorgerufen, zum Beispiel durch Blockieren der katalytisch aktiven Zentren beziehungsweise durch Verlust katalytisch aktiver Zentren durch thermische, mechanische oder chemische Prozesse. Beispielsweise kann eine Katalysatordeaktivierung oder allgemein Alterung durch Sintern der katalytisch aktiven Zentren, durch Verlust von (Edel-)Metall, durch Ablagerungen oder durch Vergiftung der aktiven Zentren verursacht werden. Die Mechanismen der Alterung/Deaktivierung sind vielfältig.

Herkömmlicherweise muss der deaktivierte Katalysator zur Regenerierung aus dem Reaktor entfernt werden. Danach liegt der Reaktor still, oder der Betrieb wird nach Einbau eines anderen Katalysators oder Umschalten auf einen bereits installierten weiteren Katalysator wieder aufgenommen. Dies führt in jedem Fall zu signifikanten Kosten. In den US-Patenten US 3,851,004 und US 2,757,128 sind Verfahren zur Hydrierung von u. a. Olefinen in Kohlenwasserstoff-Ausgangsmaterialien, sowie die Regenerierung der Katalysatoren mittels Wasserstoff offenbart. EP 0696574 und EP 0913194 offenbaren ebenfalls rutheniumhaltige Hydrierkatalysatoren und deren Regenerierung.

Die DE 196 34 880 C2 offenbart ein Verfahren zum gleichzeitigen selektiven Hydrieren von Diolefinen und Nitrilen aus einem Kohlenwasserstoff-Ausgangsmaterial. Bei diesem Verfahren wird der Katalysator, nachdem dessen Diolefin-Hydrierungsaktivität auf weniger als 50% der anfänglichen Aktivität gesunken ist, mit einem Inertgas zum Entfernen von Spuren des Kohlenwasserstoffs aus dem Katalysator und zum Schaffen eines gespülten Katalysators durchspült und in einem anschließenden Regenerierungsschritt mit Wasserstoff durchspült. Hierbei wird ein regenerierter Katalysator erzeugt, dessen Diolefin-Hydrierungsaktivität wieder mindestens 80% des anfänglichen Wertes aufweist.

Bei der Hydrierung von Aromaten unter Verwendung der beschriebenen Rutheniumkatalysatoren ist ebenfalls eine Desaktivierung zu beobachten, die bis jetzt nicht auf einfachem Weg beseitigt werden konnte.

Die Aufgabe der vorliegenden Erfindung besteht darin, ein Verfahren zum Regenerieren eines bei Hydrierungen eingesetzten Rutheniumkatalysators zur Verfügung zu stellen. Dieses soll apparativ einfach zu realisieren und in der Durchführung kostengünstig sein. Insbesondere soll sich damit eine mehrfache und vollständige Regenerierung des Katalysators erreichen lassen.

Gelöst wird die vorstehende Aufgabe durch ein Verfahren zur Regenerierung eines Rutheniumkatalysators zur Hydrierung von Aromaten, umfassend das Spülen des Katalysators mit Inertgas in einem Regenerierungsschritt, bis zum Erreichen der ursprünglichen Aktivität oder eines Teils der ursprünglichen Aktivität.

Durch diese Reaktivierung werden einerseits höhere Umsätze bedingt durch eine erhöhte Katalysatoraktivität erzielt, andererseits werden durch das erfindungsgemäße Verfahren die Katalysatorstandzeiten im Produktionsbetrieb deutlich verlängert.

Das erfindungsgemäße Verfahren eignet sich insbesondere zur Regenerierung von Ru-Katalysatoren, die in den Anmeldungen EP-A 0 814 098, EP-A 1 169 285 und DE 102 005 029 200 beschrieben und in den dort offenbarten Verfahren eingesetzt werden. Diese Katalysatoren und Verfahren seien nachstehend aufgeführt.

In der gesamten Anmeldung werden die Gruppen des Periodensystems in der CAS-Version bezeichnet.

### BEVORZUGTE KATALYSATOREN

### EP-A 0 814 098

Die nachfolgend beschriebenen Katalysatoren werden in der vorliegenden Anmeldung als "Katalysatorvariante I" bezeichnet werden.

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird.

Die Begriffe *"Makroporen"* und *"Mesoporen"* werden im Rahmen der vorliegenden Erfindung so verwendet, wie sie in Pure Appl. Chem., 45, S. 79 (1976) definiert sind, nämlich als Poren, deren Durchmesser oberhalb von 50 nm (Makroporen) oder deren Durchmesser zwischen 2 nm und 50 nm liegt (Mesoporen). *"Mikroporen"* sind ebenfalls entsprechend der obigen Literatur definiert und bezeichnen Poren mit einem Durchmesser von < 2 nm.

Der Gehalt des Aktivmetalls beträgt im Allgemeinen ungefähr 0,01 bis ungefähr 30 Gew.-%, vorzugsweise ungefähr 0,01 bis ungefähr 5 Gew.-% und insbesondere ungefähr 0,1 bis ungefähr 5 Gew.-%, jeweils bezogen auf das Gesamtgewicht des verwendeten Katalysators.

Die Metalloberfläche auf der Katalysatorvariante I beträgt dabei insgesamt vorzugsweise ungefähr 0,01 bis ungefähr 10 m²/g, weiter bevorzugt ungefähr 0,05 bis ungefähr 5 m²/g und insbesondere ungefähr 0,05 bis ungefähr 3 m²/g des Katalysators. Die Metalloberfläche wird mittels der von J. Lemaitre et al. in "Characterization of Heterogeneous Catalysts", Hrsg. Francis Delanney, Marcel Dekker, New York 1984, S. 310 - 324, beschriebenen Chemisorptionsverfahren bestimmt.

In der Katalysatorvariante I beträgt das Verhältnis der Oberflächen des/der Aktivmetalls/- metalle und des Katalysatorträgers vorzugsweise weniger als ungefähr 0,05, wobei der untere Grenzwert bei ungefähr 0,0005 liegt.

Die Katalysatorvariante I umfasst ein Trägermaterial, das makroporös ist und einen mittleren Porendurchmesser von mindestens ungefähr 50 nm, vorzugsweise mindestens ungefähr 100 nm, insbesondere mindestens ungefähr 500 nm aufweist und dessen Oberfläche nach BET bei höchstens ungefähr 30 m²/g, vorzugsweise höchstens ungefähr 15 m²/g, weiter bevorzugt höchstens ungefähr 10 m²/g, insbesondere höchstens ungefähr 5 m²/g und weiter bevorzugt höchstens ungefähr 3 m²/g liegt. Der mittlere Porendurchmesser des Trägers beträgt vorzugsweise ungefähr 100 nm bis ungefähr 200 µm, weiter bevorzugt ungefähr 500 nm bis ungefähr 50 µm. Die Oberfläche nach BET des Trägers beträgt vorzugsweise ungefähr 0,2 bis ungefähr 15 m²/g, weiter bevorzugt ungefähr 0,5 bis ungefähr 10 m²/g, insbesondere ungefähr 0,5 bis ungefähr 5 m²/g und weiter bevorzugt ungefähr 0,5 bis ungefähr 3 m²/g.

Die Oberfläche des Trägers wird bestimmt nach dem BET-Verfahren durch N₂-Adsorption, insbesondere nach DIN 66131. Die Bestimmung des mittleren Porendurchmesser und der Porengrößenverteilung erfolgt durch Hg-Porosimetrie, insbesondere nach DIN 66133.

Vorzugsweise kann die Porengrößenverteilung des Trägers annähernd bimodal sein, wobei die Porendurchmesserverteilung mit Maxima bei etwa 600 nm und etwa 20 µm bei der bimodalen Verteilung eine spezielle Ausführungsform der Erfindung darstellt.

Weiter bevorzugt ist ein Träger mit einer Oberfläche von 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Makroporen aufweisende Aktivkohle, Siliciumcarbid, Aluminiumoxid, Siliciumdioxid, Titandioxid, Zirkoniumdioxid, Magnesiumoxid, Zinkoxid oder Gemische aus zwei oder mehr davon, wobei Aluminiumoxid und Zirkoniumdioxid vorzugsweise verwendet werden.

Entsprechende Katalysatorträger bzw. Verfahren zu deren Herstellung sind offenbart in den folgenden Dokumenten:
Fundamentals of Industrial Catalytic Processes, R. J. Farrauto, C. H. Bartholomew, First Edition 1997, Seiten 16, 17, 57 bis 62, 88 bis 91, 110 bis 111; Oberlander, R. K., 1984 Aluminas for Catalysts, in Applied Industrial Catalysis, e.g. D. E. Leach, Academic Press, Vol. 3, Kapitel 4; US3,245,919; WO 93/04774; EP-A 0 243 894; Ullmann's Encyclopedia of Industrial Chemistry, 5. Ed., Vol. Al, p. 588 bis 590; VCH 1985

### EP-A 1 169 285

Die nachfolgend beschriebenen Katalysatoren werden in der vorliegenden Anmeldung als "Katalysatorvariante II" bezeichnet werden. Von dieser Variante II existieren verschiedene Untervarianten.

### Untervariante 1

Dieser Katalysator entspricht demjenigen, der vorstehend unter EP-A 0 814 089 beschrieben wurde.

Es wird weiterhin in der EP-A 1 169 285 offenbart die erfindungsgemäß verwendete Untervariante 1a, die eine bevorzugte Ausführungsform der Untervariante 1 darstellt. Die verwendbaren Trägermaterialien sind solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g aufweisen. Bevorzugt liegt der mittlere Porendurchmesser des dort verwendeten Trägers in einem Bereich von 0,1 bis 200 µm, insbesondere von 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m²/g des Trägers. Auch dieser Katalysator weist bzgl. der Porendurchmesserverteilung die bereits oben beschriebene Bimodalität mit den analogen Verteilungen und das entsprechend bevorzugte Porenvolumen auf. Weitere Details bezüglich Untervariante 1a sind der DE-A 196 04 791.9 zu entnehmen, deren Inhalt durch Bezugnahme vollständig in die vorliegende Anmeldung einbezogen wird.

### Untervariante 2

Die Untervariante 2 enthält ein oder mehrere Metalle der VIII. Nebengruppe des Periodensystems als Aktivkomponente(n) auf einem Träger, wie hierin definiert. Bevorzugt wird Ruthenium als Aktivkomponente verwendet.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J. Lemaitre et al., "Characterization of Heterogeneous Catalysts", Hrsg. Francis Delanney, Marcel Dekker, New York (1984), S. 310 - 324, beschrieben ist.

In der Untervariante 2 beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

Die bei der Untervariante 2 verwendbaren Trägermaterialien besitzen Makroporen und Mesoporen.

Dabei weisen die verwendbaren Träger eine Porenverteilung auf, dergemäß ungefähr 5 bis ungefähr 50%, vorzugsweise ungefähr 10 bis ungefähr 45%, weiter bevorzugt ungefähr 10 bis ungefähr 30% und insbesondere ungefähr 15 bis ungefähr 25% des Porenvolumens von Makroporen mit Porendurchmessern im Bereich von ungefähr 50 nm bis ungefähr 10.000 nm und ungefähr 50 bis ungefähr 95%, vorzugsweise ungefähr 55 bis ungefähr 90%, weiter bevorzugt ungefähr 70 bis ungefähr 90% und insbesondere ungefähr 75 bis ungefähr 85% des Porenvolumens von Mesoporen mit einem Porendurchmesser von ungefähr 2 bis ungefähr 50 nm gebildet werden, wobei sich jeweils die Summe der Anteile der Porenvolumina zu 100% addiert.

Das Gesamtporenvolumen der verwendeten Träger beträgt ungefähr 0,05 bis 1,5 cm³/g, vorzugsweise 0,1 bis 1,2 cm³/g und insbesondere ungefähr 0,3 bis 1,0 cm³/g. Der mittlere Porendurchmesser der erfindungsgemäß verwendeten Träger beträgt ungefähr 5 bis 20 nm, vorzugsweise ungefähr 8 bis ungefähr 15 nm und insbesondere ungefähr 9 bis ungefähr 12 nm.

Vorzugsweise beträgt die Oberfläche des Trägers ungefähr 50 bis ungefähr 500 m²/g, weiter bevorzugt ungefähr 200 bis ungefähr 350 m²/g und insbesondere ungefähr 250 bis ungefähr 300 m²/g des Trägers.

Die Oberfläche des Trägers wird nach dem BET-Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Porengrößenverteilung aufweisen, eingesetzt werden können, werden vorzugsweise Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

### DE 102 005 029 200

Die nachfolgend offenbarten Katalysatoren werden in der vorliegenden Anmeldung als Katalysatorvariante III oder auch "Schalenkatalysator" bezeichnet werden.

Gegenstand ist ein Schalenkatalysator enthaltend als Aktivmetall Ruthenium alleine oder zusammen mit mindestens einem weiteren Metall der Nebengruppen IB, VIIB oder VIII des Periodensystems der Elemente (CAS-Version), aufgebracht auf einen Träger enthaltend Siliziumdioxid als Trägermaterial.

Dieser Schalenkatalysator ist dann dadurch gekennzeichnet, dass die Menge des Aktivmetalls < 1 Gew.-%, bevorzugt 0,1 bis 0,5 Gew.-%, besonders bevorzugt 0,25 bis 0,35 Gew.-%, bezogen auf das Gesamtgewicht des Katalysators, beträgt und mindestens 60 Gew.-%, besonders bevorzugt 80 Gew.-% des Aktivmetalls, bezogen auf die Gesamtmenge des Aktivmetalls, in der Schale des Katalysators bis zu einer Eindringtiefe von 200 µm vorliegen. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Weitere Informationen bezüglich den vorstehend genannten Messverfahren und Techniken sind zum Beispiel "Spectroscopy in Catalysis" von J.W. Niemantsverdriet, VCH, 1995 offenbart.

Der Schalenkatalysator zeichnet sich dadurch aus, dass die überwiegende Menge des Aktivmetalls in der Schale bis zu einer Eindringtiefe von 200 µm, also nahe der Oberfläche des Schalenkatalysators vorliegt. Dagegen liegt im Inneren (Kern) des Katalysators keine oder nur eine sehr geringe Menge des Aktivmetalls vor. Überraschenderweise wurde gefunden, dass die Katalysatorvariante III - trotz der geringen Menge an Aktivmetall - eine sehr hohe Aktivität bei der Hydrierung von organischen Verbindungen, die hydrierbare Gruppen enthalten, insbesondere bei der Hydrierung von carbocyclischen aromatischen Gruppen, bei sehr guten Selektivitäten, aufweist. Insbesondere nimmt die Aktivität der Katalysatorvariante III über einen langen Hydrierzeitraum nicht ab.

Ganz besonders bevorzugt ist ein Schalenkatalysator, in dem kein Aktivmetall im Inneren des Katalysators nachgewiesen werden kann, d.h. Aktivmetall liegt nur in der äußersten Schale, zum Beispiel in einer Zone bis zu einer Eindringtiefe von 100 bis 200 µm, vor.

Der Schalenkatalysator zeichnet sich in einer weiteren besonders bevorzugten Ausführungsform dadurch aus, dass im (FEG)-TEM (Field Emission Gun -Transmission Electron Microscopy) mit EDXS nur in den äußersten 200 µm, bevorzugt 100 µm, ganz besonders bevorzugt 50 µm (Eindringtiefe) Aktivmetallteilchen nachweisen können. Teilchen kleiner 1 nm können nicht nachgewiesen werden.

Als Aktivmetall kann Ruthenium alleine oder zusammen mit mindestens einem weiteren Metall der Nebengruppen IB, VIIB oder VIII des Periodensystems der Elemente (CAS-Version) eingesetzt werden. Neben Ruthenium geeignete weitere Aktivmetalle sind z.B. Platin, Rhodium, Palladium, Iridium, Cobalt oder Nickel oder ein Gemisch aus zwei oder mehr davon. Unter den ebenfalls verwendbaren Metallen der Nebengruppen IB und/oder VIIB des Periodensystems der Elemente sind z.B. Kupfer und/oder Rhenium geeignet. Bevorzugt wird Ruthenium alleine als Aktivmetall oder zusammen mit Platin oder Iridium in dem Schalenkatalysator eingesetzt; ganz besonders bevorzugt wird Ruthenium alleine als Aktivmetall eingesetzt.

Der Schalenkatalysator zeigt die vorstehend erwähnte sehr hohe Aktivität bei einer geringen Beladung mit Aktivmetall, die < 1 Gew.-%, bezogen auf das Gesamtgewicht des Katalysators, beträgt. Bevorzugt beträgt die Menge des Aktivmetalls in dem erfindungsgemäßen Schalenkatalysator 0,1 bis 0,5 Gew.-%, besonders bevorzugt 0,25 bis 0,35 Gew.-%. Es wurde gefunden, dass die Eindringtiefe des Aktivmetalls in das Trägermaterial von der Beladung der Katalysatorvariante III mit Aktivmetall abhängig ist. Bereits bei einer Beladung der Katalysatorvariante III mit 1 Gew.-% oder mehr, z.B. bei einer Beladung mit 1,5 Gew.-%, ist im Inneren des Katalysators, d.h. in einer Eindringtiefe von 300 bis 1000 µm eine wesentliche Menge Aktivmetall vorhanden, die die Aktivität des Hydrierkatalysators, insbesondere die Aktivität über einen langen Hydrierzeitraum, beeinträchtigt, insbesondere bei schnellen Reaktionen, wobei Wasserstoffmangel im Inneren des Katalysators (Kern) auftreten kann.

Es liegen in dem Schalenkatalysator mindestens 60 Gew.-% des Aktivmetalls, bezogen auf die Gesamtmenge des Aktivmetalls, in der Schale des Katalysators bis zu einer Eindringtiefe von 200 µm vor. Bevorzugt liegen in dem Schalenkatalysator mindestens 80 Gew.-% des Aktivmetalls, bezogen auf die Gesamtmenge des Aktivmetalls, in der Schale des Katalysators bis zu einer Eindringtiefe von 200 µm vor. Ganz besonders bevorzugt ist ein Schalenkatalysator, in dem kein Aktivmetall im Inneren des Katalysators nachgewiesen werden kann, d.h. Aktivmetall liegt nur in der äußersten Schale, zum Beispiel in einer Zone bis zu einer Eindringtiefe von 100 bis 200 µm Zone, vor. In einer weiteren bevorzugten Ausführungsform liegen 60 Gew.-%, bevorzugt 80 Gew.-%, bezogen auf die Gesamtmenge des Aktivmetalls, in der Schale des Katalysators bis zu einer Eindringtiefe von 150 µm vor. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Zur Ermittlung der Eindringtiefe der Aktivmetallteilchen werden mehrere Katalysatorteilchen (z.B. 3, 4 oder 5) quer zur Strangachse (wenn der Katalysator in Form von Strängen vorliegt) angeschliffen. Mittels Linescans werden dann die Profile der Aktivmetall/Si Konzentrationsverhältnisse erfasst. Auf jeder Messlinie werden mehrere zum Beispiel 15 bis 20 Messpunkte in gleichen Abständen gemessen; die Messfleckgröße beträgt circa 10 µm *10 µm. Nach Integration der Aktivmetallmenge über die Tiefe kann die Häufigkeit des Aktivmetalls in einer Zone bestimmt werden.

Ganz besonders bevorzugt beträgt die Menge des Aktivmetalls, bezogen auf das Konzentrationsverhältnis von Aktivmetall zu Si, an der Oberfläche des Schalenkatalysators, 2 bis 25 %, bevorzugt 4 bis 10 %, besonders bevorzugt 4 bis 6 %, ermittelt mittels SEM EPMA - EDXS. Die Oberflächeanalyse erfolgt mittels Bereichsanalysen von Bereichen von 800 µm x 2000 µm und mit einer Informationstiefe von circa 2 µm. Die Elementzusammensetzung wird in Gew. % (normiert auf 100 %) bestimmt. Das mittlere Konzentrationsverhältnis (Aktivmetall/Si) wird über 10 Messbereiche gemittelt.

Unter der Oberfläche des Schalenkatalysators ist die äußere Schale des Katalysators bis zu einer Eindringtiefe von circa 2 µm zu verstehen. Diese Eindringtiefe entspricht der Informationstiefe bei der vorstehend erwähnten Oberflächenanalyse.

Ganz besonders bevorzugt ist ein Schalenkatalysator, worin die Menge des Aktivmetalls, bezogen auf das Gewichtsverhältnis von Aktivmetall zu Si (Gew./Gew. in %), an der Oberfläche des Schalenkatalysators 4 bis 6 % beträgt, in einer Eindringtiefe von 50 µm 1,5 bis 3 % und im Bereich von 50 bis 150 µm Eindringtiefe 0,5 bis 2 %, ermittelt mittels SEM EPMA (EDXS), beträgt. Die genannten Werte stellen gemittelte Werte dar.

Des Weiteren nimmt die Größe der Aktivmetallteilchen bevorzugt mit zunehmender Eindringtiefe ab, ermittelt mittels (FEG)-TEM-Analyse.

Das Aktivmetall liegt in dem Schalenkatalysator bevorzugt teilweise oder vollständig kristallin vor. In bevorzugten Fällen kann in der Schale des Schalenkatalysators mittels SAD (Selected Area Diffraction) oder XRD (X-Ray Diffraction) feinstkristallines Aktivmetall nachgewiesen werden.

Der Schalenkatalysator kann zusätzlich Erdalkalimetallionen (M²⁺), also M = Be, Mg, Ca, Sr und/oder Ba, insbesondere Mg und/oder Ca, ganz besonders Mg enthalten. Der Gehalt an Erdalkalimetallion/en (M²⁺) im Katalysator beträgt bevorzugt 0,01 bis 1 Gew.-%, insbesondere 0,05 bis 0,5 Gew.-%, ganz besonders 0,1 bis 0,25 Gew.-%, jeweils bezogen auf das Gewicht des Siliziumdioxid-Trägermaterials.

Ein wesentlicher Bestandteil der Katalysatorvariante III ist das Trägermaterial auf Basis von Siliziumdioxid, im Allgemeinen amorphem Siliziumdioxid. Unter dem Begriff "amorph" versteht man in diesem Zusammenhang, dass der Anteil kristalliner Siliziumdioxid-Phasen weniger als 10 Gew.-% des Trägermaterials ausmacht. Die zur Herstellung der Katalysatoren verwendeten Trägermaterialien können allerdings Überstrukturen aufweisen, die durch regelmäßige Anordnung von Poren im Trägermaterial gebildet werden.

Als Trägermaterialien kommen grundsätzlich amorphe Siliziumdioxid-Typen in Betracht, die wenigstens zu 90 Gew.-% aus Siliziumdioxid bestehen, wobei die verbleibenden 10 Gew.-%, vorzugsweise nicht mehr als 5 Gew.-%, des Trägermaterials auch ein anderes oxidisches Material sein können, z.B. MgO, CaO, TiO₂, ZrO₂, Fe₂O₃ und/oder Alkalimetalloxid.

In einer bevorzugten Ausführungsform der Erfindung ist das Trägermaterial halogenfrei, insbesondere chlorfrei, d. h. der Gehalt an Halogen im Trägermaterial beträgt weniger als 500 Gew.-ppm, z.B. im Bereich von 0 bis 400 Gew.-ppm. Somit ist ein Schalenkatalysator bevorzugt, der weniger als 0,05 Gew.-% Halogenid (ionenchromatographisch bestimmt), bezogen auf das Gesamtgewicht des Katalysators, enthält.

Bevorzugt sind Trägermaterialien, die eine spezifische Oberfläche im Bereich von 30 bis 700 m²/g, vorzugsweise 30 bis 450 m²/g, (BET-Oberfläche nach DIN 66131) aufweisen.

Geeignete amorphe Trägermaterialien auf Basis von Siliziumdioxid sind dem Fachmann geläufig und kommerziell erhältlich (siehe z.B. O.W. Flörke, "Silica" in Ullmann's Encyclopedia of Industrial Chemistry 6th Edition on CD-ROM). Sie können sowohl natürlichen Ursprungs als auch künstlich hergestellt worden sein. Beispiele für geeignete amorphe Trägermaterialien auf Basis von Siliziumdioxid sind Kieselgele, Kieselgur, pyrogene Kieselsäuren und Fällungskieselsäuren. In einer bevorzugten Ausführungsform der Erfindung weisen die Katalysatoren Kieselgele als Trägermaterialien auf.

Je nach Ausgestaltung der Erfindung kann das Trägermaterial unterschiedliche Gestalt aufweisen. Sofern das Verfahren, in dem die Schalenkatalysatoren eingesetzt werden, als Suspensionsverfahren ausgestaltet ist, wird man zur Herstellung der Katalysatoren üblicherweise das Trägermaterial in Form eines feinteiligen Pulvers einsetzen. Vorzugsweise weist das Pulver Teilchengrößen im Bereich von 1 bis 200 µm insbesondere 1 bis 100 µm auf. Bei Einsatz des erfindungsgemäßen Schalenkatalysators in Katalysatorfestbetten verwendet man üblicherweise Formkörper aus dem Trägermaterial, die z.B. durch Extrudieren, Strangpressen oder Tablettieren erhältlich sind und die z.B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 0,5 mm bis 25 mm. Häufig werden Katalysatorstränge mit Strangdurchmessern von 1,0 bis 5 mm und Stranglängen von 2 bis 25 mm eingesetzt. Mit kleineren Strängen können im Allgemeinen höhere Aktivitäten erzielt werden; jedoch zeigen diese oft keine ausreichende mechanische Stabilität im Hydrierverfahren. Daher werden ganz besonders bevorzugt Stränge mit Strangdurchmessern im Bereich von 1,5 bis 3 mm eingesetzt.

### BEVORZUGTE VERFAHREN UNTER EINSATZ DER KATALYSATOREN

Die vorstehend beschriebenen Katalysatoren (Katalysatorvarianten I, II und III und genannte Untervarianten) werden bevorzugt als Hydrierkatalysator eingesetzt. Sie sind insbesondere zur Hydrierung von organischen Verbindungen geeignet, die hydrierbare Gruppen enthalten. Bei den hydrierbaren Gruppen kann es sich um Gruppen handeln, die die folgenden Struktureinheiten aufweisen: C-C-Doppelbindungen, C-C-Dreifachbindungen, aromatische Gruppen, C-N-Doppelbindungen, C-N-Dreifachbindungen, C-O-Doppelbindungen, N-O-Doppelbindungen, NO₂-Gruppen, wobei die Gruppen auch in Polymeren oder cyclischen Strukturen enthalten sein können, z.B. in ungesättigten Heterocyclen. Die hydrierbaren Gruppen können jeweils einzeln oder mehrfach in den organischen Verbindungen vorkommen. Es ist auch möglich, dass die organischen Verbindungen zwei oder mehr verschiedene der genannten hydrierbaren Gruppen aufweisen. Je nach Hydrierbedingungen ist es in dem letzten Fall möglich, dass nur eine oder mehrere der hydrierbaren Gruppen hydriert werden. Es ist auch möglich, dass eine funktionelle Gruppe vorhanden ist, die intakt bleibt. Dies ist etwa der Fall bei -OH oder NH-Funktionen, die sich an einem aromatischen Rest befinden, der zu der entsprechenden carbocyclischen Gruppe hydriert wird, unter Erhalt der beschriebenen funktionellen Gruppe.

Bevorzugt werden die oben beschriebenen Katalysatoren eingesetzt zur Hydrierung einer carbocyclischen aromatischen Gruppe zu der entsprechenden carbocyclischen aliphatischen Gruppe. Besonders bevorzugt erfolgt dabei eine vollständige Hydrierung der aromatischen Gruppe, wobei unter vollständiger Hydrierung ein Umsatz der zu hydrierenden Verbindung von im Allgemeinen > 98 %, bevorzugt > 99 %, besonders bevorzugt > 99,5 %, ganz besonders bevorzugt > 99,9 %, insbesondere >99,99 % und speziell >99,995 % zu verstehen ist.

Bei einem Einsatz der oben beschriebenen Katalysatorvarianten I, II und III zur Hydrierung von Benzol zu Cyclohexan werden somit die typischen Cyclohexan-Spezifikationen, die einen Benzol-Restgehalt von < 100 ppm fordern (das entspricht einem Benzol-Umsatz von >99,99%), eingehalten. Bevorzugt ist der Benzol-Umsatz bei einer Hydrierung von Benzol mit dem erfindungsgemäßen Schalenkatalysator > 99,995%.

Bei einem Einsatz der Katalysatorvarianten I, II und/oder III zur Hydrierung von aromatischen Dicarbonsäureestern, insbesondere Phthalsäureestern, zu den entsprechenden Dialkylcyclohexandicarboxylaten werden somit ebenfalls die typischen Spezifikationen, die einen Restgehalt des aromatischen Dicarbonsäureesters, insbesondere Phthalsäureester-Restgehalt, von < 100 ppm fordern (das entspricht einem Umsatz von > 99,99%), eingehalten. Bevorzugt ist der Umsatz bei einer Hydrierung von aromatischen Dicarbonsäureestern, insbesondere Phthalsäureestern, mit dem erfindungsgemäßen Schalenkatalysator > 99,995%.

Ein weiterer Gegenstand der vorliegenden Anmeldung ist daher ein Verfahren zur Hydrierung einer organischen Verbindung, die hydrierbare Gruppen enthält, bevorzugt zur Hydrierung einer carbocyclischen aromatischen Gruppe zu der entsprechenden carbocyclischen aliphatischen Gruppe, wobei bei Hydrierung eventuell vorhandene funktionelle Gruppen, beispielsweise -OH oder NH-Gruppen intakt bleiben, und die neben dem Hydrierschritt einen Regenerierungsschritt aufweist.

Die carbocyclische aromatische Gruppe ist bevorzugt Teil eines aromatischen Kohlenwasserstoffs, der die folgende allgemeine Formel aufweist:

(A)-(B)ₙ

worin die Symbole die folgende Bedeutung haben:
- A: unabhängig Aryl oder Heteroaryl, bevorzugt ist A ausgewählt aus Phenyl, Diphenyl, Benzyl, Dibenzyl, Naphthyl, Anthracen, Pyridyl und Chinolin, besonders bevorzugt ist A Phenyl oder Naphthyl,
- n: eine Zahl von 0 bis 5, bevorzugt 0 bis 4, besonders bevorzugt 0 bis 3, insbesondere in dem Fall, wenn A ein 6-gliedriger Aryl- oder Heteroaryl-Ring ist; für den Fall, dass A ein 5-gliedriger Aryl- oder Heteroaryl-Ring ist, ist n bevorzugt 0 bis 4; unabhängig von der Ringgröße ist n besonders bevorzugt 0 bis 3, ganz besonders bevorzugt 0 bis 2 und insbesondere 0 bis 1; dabei tragen die übrigen, keine Substituenten B tragenden Kohlenwasserstoff- oder Heteroatome von A Wasserstoffatome oder gegebenenfalls keinen Substituenten;
- B: ist unabhängig voneinander ausgewählt aus der Gruppe bestehend aus Alkyl, Alkenyl, Alkinyl, substituiertem Alkyl, substituiertem Alkenyl, substituiertem Alkinyl, Heteroalkyl, substiuiertem Heteroalkyl, Heteroalkenyl, substituiertem Heteroalkenyl, Heteroalkinyl, substituiertem Heteroalkinyl, Cycloalkyl, Cycloalkenyl, substituiertem Cycloalkyl, substituiertem Cycloalkenyl, COOR, wobei R H, Alkyl, substituiertes Alkyl, Cycloalkyl, substituiertes Cycloalkyl, Aryl oder substituiertes Aryl bedeutet, Halogen, Hydroxy, Alkoxy, Aryloxy, Carbonyl, Amino, Amido und Phosphino; bevorzugt ist B unabhängig voneinander ausgewählt aus C₁₋₆-Alkyl, C₁₋₆-Alkenyl, C₁₋₆-Alkinyl, C₃₋₈-Cycloalkyl, C₃₋₈-Cycloalkenyl, COOR, wobei R H oder C₁₋₁₂-Alkyl bedeutet, Hydroxy, Alkoxy, Aryloxy, Amino und Amido, besonders bevorzugt ist B unabhängig voneinander C₁₋₆-Alkyl,COOR, wobei R H oder C₁₋₁₂-Alkyl bedeutet, Amino, Hydroxy oder Alkoxy.

Der Ausdruck unabhängig voneinander bedeutet, dass, wenn n 2 oder größer ist, die Substituenten B gleiche oder verschiedene Reste aus den genannten Gruppen sein können.

Unter Alkyl sind gemäß der vorliegenden Anmeldung verzweigte oder lineare, gesättigte acyclische Kohlenwasserstoff-Reste zu verstehen, beispielsweise Alkyl-Reste mit 1 bis 50 Kohlenstoffatomen, bevorzugt mit 1 bis 20 Kohlenstoffatomen.

In der oben erwähnten Gruppe COOR bedeutet R H oder verzweigtes oder lineares Alkyl, bevorzugt H oder C₁₋₁₂-Alkyl. Bevorzugte Alkylgruppen sind C₄₋₁₀-Alkylgruppen, besonders bevorzugt C₈₋₁₀-Alkylgruppen. Diese können verzweigt oder unverzweigt sein und sind bevorzugt verzweigt. Bei den Alkylgruppen mit mehr als drei Kohlenstoffatomen kann es sich um Isomerengemische verschiedener Alkylgruppen mit derselben Kohlenstoffzahl handeln.

Ein Beispiel ist eine C₉-Alkylgruppe, wobei es sich um eine Isononylgruppe handeln kann, also um ein Isomerengemisch verschiedener C₉-Alkylgruppen. Gleiches gilt z.B. für eine C₈-Alkylgruppe. Solche Isomerengemische werden ausgehend von den den Alkylgruppen entsprechenden Alkoholen erhalten, die aufgrund ihres - dem Fachmann bekannten - Herstellungsverfahrens als Isomerengemische anfallen.

Unter Alkenyl sind gemäß der vorliegenden Anmeldung verzweigte oder unverzweigte acyclische Kohlenwasserstoff-Reste zu verstehen, die mindestens eine Kohlenstoff-Kohlenstoff-Doppelbindung aufweisen. Geeignete Alkenyl-Reste sind beispielsweise 2-Propenyl, Vinyl, usw.. Bevorzugt weisen die Alkenyl-Reste 2 bis 50 Kohlenstoffatome, besonders bevorzugt 2 bis 20 Kohlenstoffatome, ganz besonders bevorzugt 2 bis 6 Kohlenstoffatome und insbesondere 2 bis 3 Kohlenstoffatome auf. Weiterhin sind unter dem Begriff Alkenyl solche Reste zu verstehen, die entweder eine cis- oder eine trans-Orientierung (alternativ E- oder Z-Orientierung) aufweisen.

Unter Alkinyl sind gemäß der vorliegenden Anmeldung verzweigte oder unverzweigte acyclische Kohlenwasserstoff-Reste zu verstehen, die mindestens eine Kohlenstoff-Kohlenstoff-Dreifachbindung aufweisen. Vorzugsweise weisen die Alkinyl-Reste 2 bis 50 Kohlenstoffatome, besonders bevorzugt 2 bis 20 Kohlenstoffatome, ganz besonders bevorzugt 1 bis 6 Kohlenstoffatome und insbesondere 2 bis 3 Kohlenstoffatome auf.

Unter substituiertem Alkyl, substituiertem Alkenyl und substituiertem Alkinyl sind Alkyl-Alkenyl- und Alkinyl-Reste zu verstehen, worin ein oder mehrere Wasserstoffatome, die an ein Kohlenstoffatom dieser Reste gebunden sind, durch eine andere Gruppe ersetzt sind. Beispiele für solche andere Gruppen sind Heteroatome, Halogen, Aryl, substituiertes Aryl, Cycloalkyl, Cycloalkenyl, substituiertes Cycloalkyl, substituiertes Cycloalkenyl und Kombinationen davon. Beispiele für geeignete substituierte Alkyl-Reste sind Benzyl, Trifluormethyl u. a.

Unter den Begriffen Heteroalkyl, Heteroalkenyl und Heteroalkinyl sind Alkyl- Alkenyl- und Alkinyl-Reste zu verstehen, worin ein oder mehrere der Kohlenstoffatome in der Kohlenstoffkette durch ein Heteroatom ausgewählt aus N und O ersetzt sind. Die Bindung zwischen dem Heteroatom und einem weiteren Kohlenstoffatom kann dabei gesättigt oder gegebenenfalls ungesättigt sein.

Unter Cycloalkyl sind gemäß der vorliegenden Anmeldung gesättigte cyclische nicht-aromatische Kohlenwasserstoff-Reste zu verstehen, die aus einem einzigen Ring oder mehreren kondensierten Ringen aufgebaut sind. Geeignete Cycloalkyl-Reste sind beispielsweise Cyclopentyl, Cyclohexyl, Cyclooctanyl, Bicyclooctyl usw.. Bevorzugt weisen die Cycloalkyl-Reste zwischen 3 und 50 Kohlenstoffatome, besonders bevorzugt zwischen 3 und 20 Kohlenstoffatome, ganz besonders bevorzugt zwischen 3 und 8 Kohlenstoffatome und insbesondere zwischen 3 und 6 Kohlenstoffatome auf.

Unter Cycloalkenyl sind gemäß der vorliegenden Anmeldung teilweise ungesättigte, cyclische nicht-aromatische Kohlenwasserstoff-Reste zu verstehen, die einen einzigen oder mehrere kondensierte Ringe aufweisen. Geeignete Cycloalkenyl-Reste sind beispielsweise Cyclopentenyl, Cyclohexenyl, Cyclooctenyl usw.. Bevorzugt weisen die Cycloalkenyl-Reste 3 bis 50 Kohlenstoffatome, besonders bevorzugt 3 bis 20 Kohlenstoffatome, ganz besonders bevorzugt 3 bis 8 Kohlenstoffatome und insbesondere 3 bis 6 Kohlenstoffatome auf.

Substituierte Cycloalkyl- und substituierte Cycloalkenyl-Reste sind Cycloalkyl- und Cycloalkenyl-Reste, worin ein oder mehrere Wasserstoffatome eines beliebigen Kohlenstoffatoms des Kohlenstoffrings durch eine andere Gruppe ersetzt sind. Solche andere Gruppen sind beispielsweise Halogen, Alkyl, Alkenyl, Alkinyl, substituiertes Alkyl, substituiertes Alkenyl, substituiertes Alkinyl, Aryl, substituiertes Aryl, Cycloalkyl, Cycloalkenyl, substituiertes Cycloalkyl, substituiertes Cycloalkenyl, ein aliphatischer heterocyclischer Rest, ein substituierter aliphatischer heterocyclischer Rest, Heteroaryl, substituiertes Heteroaryl, Alkoxy, Aryloxy, Boryl, Phosphino, Amino, Silyl, und Kombinationen davon. Beispiele für substituierte Cycloalkyl und Cycloalkenyl-Reste sind 4-Dimethylaminocyclohexyl, 4,5-Dibromocyclohept-4-enyl.

Unter Aryl sind im Sinne der vorliegenden Anmeldung aromatische Reste zu verstehen, die einen einzelnen aromatischen Ring oder mehrere aromatische Ringe aufweisen, die kondensiert sind, über eine kovalente Bindung verknüpft sind oder durch eine geeignete Einheit, z. B. eine Methylen- oder Ethylen-Einheit verknüpft sind. Solche geeignete Einheiten können auch Carbonyl-Einheiten, wie in Benzophenol, oder Sauerstoff-Einheiten, wie in Diphenylether, oder Stickstoff-Einheiten, wie in Diphenylamin, sein. Der aromatische Ring, bzw. die aromatischen Ringe, sind beispielsweise Phenyl, Naphthyl, Diphenyl, Diphenylether, Diphenylamin und Benzophenon. Bevorzugt weisen die Aryl-Reste 6 bis 50 Kohlenstoffatome, besonders bevorzugt 6 bis 20 Kohlenstoffatome, ganz besonders bevorzugt 6 bis 8 Kohlenstoffatome auf.

Substituierte Aryl-Reste sind Aryl-Reste, in denen ein oder mehrere Wasserstoffatome, die an Kohlenstoffatome des Aryl-Rests gebunden sind, durch eine oder mehrere andere Gruppen ersetzt sind. Geeignete andere Gruppen sind Alkyl, Alkenyl, Alkinyl, substituiertes Alkyl, substituiertes Alkenyl, substituiertes Alkinyl, Cycloalkyl, Cycloalkenyl, substituiertes Cycloalkyl, substituiertes Cycloalkenyl, Heterocyclo, substituiertes Heterocyclo, Halogen, halogensubstituiertes Alkyl (z. B. CF₃), Hydroxy, Amino, Phosphino, Alkoxy, und sowohl gesättigte als auch ungesättigte cyclische Kohlenwasserstoffe, die an den aromatischen Ring bzw. an die aromatischen Ringe kondensiert sein können oder durch eine Bindung verknüpft sein können, oder über eine geeignete Gruppe miteinander verknüpft sein können. Geeignete Gruppen sind bereits vorstehend erwähnt.

Unter Heteroaryl-Resten sind solche Aryl-Reste zu verstehen, in denen einer oder mehrere der Kohlenstoffatome des aromatischen Rings des Aryl-Rests durch ein Heteroatom ausgewählt aus N und O ersetzt ist/sind.

Unter substituierten Heteroaryl-Resten sind solche substituierten Aryl-Reste zu verstehen, in denen einer oder mehrere der Kohlenstoffatome des aromatischen Rings des substituierten Aryl-Rests durch ein Heteroatom ausgewählt aus N und O ersetzt ist/sind.

Unter Heterocyclo ist gemäß der vorliegenden Anmeldung ein gesättigter, teilweise ungesättigter oder ungesättigter cyclischer Rest zu verstehen, worin ein oder mehrere Kohlenstoffatome des Rests durch ein Heteroatom, z. B. N und O ersetzt sind (unter den Begriff "Heterocyclo" fallen auch die vorstehend genannten Heteroaryl-Reste). Beispiele für Heterocyclo-Reste sind Piperazinyl, Morpholinyl, Tetrahydropyranyl, Tetrahydrofuranyl, Piperidinyl, Pyrolidinyl, Oxazolinyl, Pyridyl, Pyrazyl, Pyridazyl, Pyrimidyl.

Substituierte Heterocyclo-Reste sind solche Heterocyclo-Reste, in denen ein oder mehrere Wasserstoffatome, die an eines der Ringatome gebunden sind, durch eine andere Gruppe ersetzt sind. Geeignete andere Gruppen sind Halogen, Alkyl, substituiertes Alkyl, Aryl, substituiertes Aryl, Heteroaryl, substituiertes Heteroaryl, Alkoxy, Aryloxy, Boryl, Phosphino, Amino, Silyl, und Kombinationen davon.

Unter Alkoxy-Resten sind Reste der allgemeinen Formel -OZ¹ zu verstehen, worin Z¹ ausgewählt ist aus Alkyl, substituiertem Alkyl, Cycloalkyl, substituiertem Cycloalkyl, Heterocycloalkyl, substituiertem Heterocycloalkyl, Silyl und Kombinationen davon. Geeignete AlkoxyReste sind beispielsweise Methoxy, Ethoxy, Benzyloxy, t-Butoxy usw.. Unter dem Begriff Aryloxy sind solche Reste der allgemeinen Formel -OZ¹ zu verstehen, worin Z¹ ausgewählt ist aus Aryl, substituiertem Aryl, Heteroaryl, substituiertem Heteroaryl und Kombinationen davon. Geeignete Aryloxy-Reste sind Phenoxy, substituiertes Phenoxy, 2-Pyridinoxy, 8-Chinolinoxy u.a..

In einer bevorzugten Ausführungsform ist A Phenyl, n 0 bis 3 und B C₁₋₆-Alkyl, COOR, wobei R H oder C₁₋₁₂-Alkyl bedeutet, Amino, Hydroxy oder Alkoxy. Das erfindungsgemäße Hydrierverfahren erfolgt dabei bevorzugt in der Weise, dass die Phenylgruppe vollständig zu der entsprechenden Cyclohexylgruppe hydriert wird.

Bevorzugte Verbindungen, die zu ihren entsprechenden Cyclohexylderivaten hydriert werden sind nachfolgend genannt.

In einer bevorzugten Ausführungsform des Hydrierverfahrens ist der aromatische Kohlenwasserstoff ausgewählt aus der Gruppe bestehend aus Benzol und alkylsubstituierten Benzolen wie Toluol, Ethylbenzol, Xylol (o-, m-, p- oder Isomerengemisch) und Mesitylol (1,2,4 oder 1,3,5 oder Isomerengemisch). In dem erfindungsgemäßen Verfahren werden somit bevorzugt Benzol zu Cyclohexan und die alkylsubstituierten Benzole wie Toluol, Ethylbenzol, Xylol und Mesitylol zu alkylsubstituierten Cyclohexanen wie Methylcyclohexan, Ethylcyclohexan, Dimethylcyclohexan und Trimethylcyclohexan hydriert. Es können auch beliebige Mischungen der vorstehend genannten aromatischen Kohlenwasserstoffe zu Mischungen der entsprechenden Cyclohexane hydriert werden. Beispielsweise können beliebige Mischungen enthaltend zwei oder drei Verbindungen ausgewählt aus Benzol, Toluol und Xylol zu Mischungen enthaltend zwei oder drei Verbindungen ausgewählt aus Cyclohexan, Methylcyclohexan und Dimethylcyclohexan hydriert werden.

In einer weiteren bevorzugten Ausführungsform der Erfindung ist der aromatische Kohlenwasserstoff ausgewählt aus Cumol, Diphenylmethan, Tri-, Tetra-, Penta- und Hexabenzolen, Triphenylmethan, alkylsubstituierten Naphthalinen, Naphthalin, alkylsubstituierten Anthracenen, Anthracen, alkylsubstituierten Tetralinen, Tetralin und Diisononylphthalat. Ein bevorzugtes Beispiel ist die Hydrierung von Naphthalin zu Tetralin oder Decalin.

In einer weiteren bevorzugten Ausführungsform des Hydrierverfahrens ist der aromatische Kohlenwasserstoff ausgewählt aus der Gruppe bestehend aus Phenol, alkylsubstituierten Phenolen wie 4-tert.-Butylphenol und 4-Nonylphenol, Bis(p-hydroxyphenyl)methan und Bis(p-hydroxyphenyl)dimethylmethan. In dem erfindungsgemäßen Verfahren werden somit bevorzugt Phenol zu Cyclohexanol, die alkylsubstituierten Phenole wie 4-tert.-Butylphenol und 4-Nonylphenol, zu alkylsubstituierten Cyclohexanolen wie 4-tert.-Butylcyclohexanol und 4-Nonylcyclohexanol, Bis(p-hydroxyphenyl)methan zu Bis(p-hydroxycyclohexyl)methan und Bis(p-hydroxyphenyl)dimethylmethan zu Bis(p-hydroxy cyclohexyl)dimethylmethan hydriert. In einer Variante dieser bevorzugten Ausführungsform ist die Hydroxyfunktion des Phenols verethert, vorzugsweise zu Alkoxyphenolen. Vorzugsweise wird ein Ethoxyphenol eingesetzt, das unsubstituiert sein oder Substituenten tragen kann.

In einer weiteren bevorzugten Ausführungsform des erfindungsgemäßen Hydrierverfahrens ist der aromatische Kohlenwasserstoff ausgewählt aus der Gruppe bestehend aus Anilin, alkylsubstituiertem Anilin, N,N-Dialkylanilin, Diaminobenzol, Bis(p-aminophenyl)methan und Bis(p-aminotolyl)methan. In dem erfindungsgemäßen Verfahren werden somit bevorzugt Anilin zu Cyclohexylamin, alkylsubstituiertes Anilin zu alkylsubstituiertem Cyclohexylamin, N,N-Dialkylanilin zu N,N-Dialkylcyclohexylamin, 2,6-Dimethylanilin zu 2,6-Dicyclohexylamin, Diaminobenzol zu Diaminocyclohexan, Bis(p-aminophenyl)methan zu Bis(p-aminocyclohexyl)methan, (2-Aminophenyl)-(4-aminophenyl)methan zu (2-Aminocyclohexyl)-(4-aminocyclohexyl)methan und Bis(p-aminotolyl)methan zu Bis(p-aminomethylcyclohexyl)methan hydriert.

In einer weiteren bevorzugten Ausführungsform des erfindungsgemäßen Hydrierverfahrens ist der aromatische Kohlenwasserstoff ausgewählt ist aus der Gruppe bestehend aus aromatischen Carbonsäuren wie Phthalsäure und aromatischen Carbonsäureestern wie C₁₋₁₂-Alkylestern der Phthalsäure, wobei die C₁₋₁₂-Alkylreste linear oder verzweigt sein können, z.B. Dimethylphthalat, Di-2-propylheptylphthalat, Di-2-ethylhexylphthalat, Dioctylphthalat, Diisononylphthalat. In dem erfindungsgemäßen Verfahren werden somit bevorzugt aromatische Carbonsäuren wie Phthalsäure zu cycloaliphatischen Carbonsäuren wie Tetrahydrophthalsäure und aromatische Carbonsäureester wie C₁₋₁₂-Alkylester der Phthalsäure, zu aliphatischen Carbonsäureestern wie C₁₋₁₂-Alkylestern der Tetrahydrophthalsäure z.B. Dimethylphthalat zu Dimethylcyclohexandicarboxylat, Di-2-propylheptylphthalat zu Di-2-propylheptylcyclohexandicarboxylat, Di-2-ethylhexylphthalat zu Di-2-ethylhexylcyclohexandicarboxylat, Dioctylphthalat zu Dioctylcyclohexandicarboxylat und Diisononylphthalat zu Diisononylcyclohexan-dicarboxylat, hydriert.

In einer weiteren gegebenenfalls möglichen Ausführungsform betrifft die vorliegende Anmeldung ein Verfahren zur Hydrierung von Aldehyden zu den entsprechenden Alkoholen. Bevorzugte Aldehyde sind Mono- und Disaccharide wie Glucose, Lactose und Xylose. Die Mono- und Disaccharide werden zu den entsprechenden Zuckeralkoholen hydriert, z.B. wird Glukose zu Sorbitol, Lactose zu Lactitol und Xylose zu Xylitol hydriert.

Geeignete Mono- und Disaccharide und geeignete Hydrierbedingungen sind z.B. in DE-A 101 28 205 offenbart, wobei anstelle des in DE-A 101 28 205 offenbarten Katalysators der Schalenkatalysator gemäß der vorliegenden Erfindung eingesetzt wird.

Das Hydrierverfahren kann in der Flüssigphase oder in der Gasphase durchgeführt werden. Bevorzugt wird das erfindungsgemäße Hydrierverfahren in der Flüssigphase durchgeführt.

Das Hydrierverfahren kann in Abwesenheit eines Lösungs- oder Verdünnungsmittels oder Gegenwart eines Lösungs- oder Verdünnungsmittels durchgeführt werden, d.h. es ist nicht erforderlich, die Hydrierung in Lösung durchzuführen.

Als Lösungs- oder Verdünnungsmittel kann jedes geeignete Lösungsmittel- oder Verdünnungsmittel eingesetzt werden. Als Lösungs- oder Verdünnungsmittel kommen grundsätzlich solche in Betracht, die die zu hydrierende organische Verbindung möglichst vollständig zu lösen vermögen oder sich mit dieser vollständig mischen und die unter den Hydrierungsbedingungen inert sind, d.h. nicht hydriert werden.

Beispiele für geeignete Lösungsmittel sind cyclische und acyclische Ether, z.B. Tetrahydrofuran, Dioxan, Methyl-tert.-butylether, Dimethoxyethan, Dimethoxypropan, Dimethyldiethylenglykol, aliphatische Alkohole wie Methanol, Ethanol, n- oder Isopropanol, n-, 2-, iso- oder tert.-Butanol, Carbonsäureester wie Essigsäuremethylester, Essigsäureethylester, Essigsäurepropylester oder Essigsäurebutylester, sowie aliphatische Etheralkohole wie Methoxypropanol und cycloaliphatische Verbindungen wie Cyclohexan, Methylcyclohexan und Dimethylcyclohexan.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 3 bis 70 gew.-%igen Lösung der zur Hydrierung vorgesehenen organischen Verbindung führen. Der Einsatz eines Verdünnungsmittels ist vorteilhaft, um eine zu starke Wärmetönung im Hydrierverfahren zu vermeiden. Eine zu starke Wärmetönung kann zu einer Deaktivierung des Katalysators führen und ist daher unerwünscht. Daher ist in dem erfindungsgemäßen Hydrierverfahren eine sorgfältige Temperaturkontrolle sinnvoll. Geeignete Hydriertemperaturen sind nachstehend genannt.

Bei Verwendung eines Lösungsmittels wird im Rahmen des erfindungsgemäßen Verfahrens besonders bevorzugt das bei der Hydrierung gebildete Produkt, also bevorzugt der oder die jeweilige(n) Cycloaliphat(en) als Lösungsmittel eingesetzt, gegebenenfalls neben anderen Lösungs- oder Verdünnungsmitteln. In jedem Fall kann ein Teil des im Verfahren gebildeten Produkts dem noch zu hydrierenden Aromaten beigemischt werden. Bei der Hydrierung von Benzol wird somit in einer besonders bevorzugten Ausführungsform Cyclohexan als Lösungsmittel verwendet. Bei der Hydrierung von Phthalaten werden bevorzugt die entsprechenden Dialkylcyclohexandicarbonsäureester als Lösungsmittel eingesetzt.

Bezogen auf das Gewicht der zur Hydrierung vorgesehenen organischen Verbindung wird vorzugsweise die 1 bis 30-fache, besonders bevorzugt die 5 bis 20-fache, insbesondere die 5 bis 10-fache Menge an Produkt als Lösungs- oder Verdünnungsmittel zugemischt. Insbesondere betrifft die vorliegende Erfindung eine Hydrierung der hier in Rede stehenden Art, wobei Benzol in Gegenwart des erfindungsgemäßen Katalysators zu Cyclohexan hydriert wird.

Die eigentliche Hydrierung erfolgt üblicherweise in Analogie zu den bekannten Hydrierverfahren zur Hydrierung von organischen Verbindungen, die hydrierbare Gruppen aufweisen, bevorzugt zur Hydrierung einer carbocyclischen aromatischen Gruppe zu der entsprechenden carbocyclischen aliphatischen Gruppe, wie sie im eingangs genannten Stand der Technik beschrieben werden. Hierzu wird die organische Verbindung als flüssige Phase oder Gasphase, bevorzugt als flüssige Phase, mit dem Katalysator in Gegenwart von Wasserstoff in Kontakt gebracht. Die flüssige Phase kann man über eine KatalysatorSuspension (Suspensions-Fahrweise) oder ein Katalysator-Festbett (Festbettfahrweise) geleitet werden.

Die Hydrierung kann sowohl kontinuierlich als auch diskontinuierlich ausgestaltet werden, wobei die kontinuierliche Verfahrensdurchführung bevorzugt ist. Vorzugsweise führt man das erfindungsgemäße Verfahren in Rieselreaktoren oder in gefluteter Fahrweise nach der Festbettfahrweise durch. Der Wasserstoff kann dabei sowohl im Gleichstrom mit der Lösung des zu hydrierenden Edukts als auch im Gegenstrom über den Katalysator geleitet werden.

Geeignete Apparaturen zur Durchführung einer Hydrierung nach der Hydrierung am Katalysatorfließbett und am Katalysatorfestbett sind aus dem Stand der Technik bekannt, z.B. aus Ullmanns Enzyklopädie der Technischen Chemie, 4. Auflage, Band 13, S. 135 ff., sowie aus P. N. Rylander, "Hydrogenation and Dehydrogenation" in Ullmann's Encyclopedia of Industrial Chemistry, 5th ed. on CD-ROM.

Die Hydrierung kann sowohl bei Wasserstoffnormaldruck als auch bei erhöhtem Wasserstoffdruck, z.B. bei einem Wasserstoffabsolutdruck von wenigstens 1,1 bar, vorzugsweise wenigstens 2 bar durchgeführt werden. Im Allgemeinen wird der Wasserstoffabsolutdruck einen Wert von 325 bar und vorzugsweise 300 bar nicht überschreiten. Besonders bevorzugt liegt der Wasserstoffabsolutdruck im Bereich von 1,1 bis 300 bar. Die Hydrierung von Benzol erfolgt z.B. bei einem Wasserstoffdruck von im Allgemeinen ≤ 50 bar, bevorzugt 10 bar bis 45 bar, besonders bevorzugt 15 bis 40 bar, meist bevorzugt 18 bis 38 bar.

Die Reaktionstemperaturen betragen im Verfahren im Allgemeinen wenigstens 30°C und werden häufig einen Wert von 250 °C nicht überschreiten. Bevorzugt führt man das Hydrierverfahren bei Temperaturen im Bereich von 50 bis 200 °C, besonders bevorzugt 70 bis 180°C, und ganz besonders bevorzugt im Bereich von 80 bis 160°C durch. Die Hydrierung von Benzol erfolgt z.B. bei Temperaturen im Bereich von im Allgemeinen 75°C bis 170°C, bevorzugt 80°C bis 160°C.

Als Reaktionsgase kommen neben Wasserstoff auch wasserstoffhaltige Gase in Betracht, die keine Katalysatorgifte wie Kohlenmonoxid oder schwefelhaltige Gase wie H₂S oder COS enthalten, z.B. Mischungen von Wasserstoff mit Inertgasen wie Stickstoff oder Reformer-Abgase, die üblicherweise noch flüchtige Kohlenwasserstoffe enthalten. Bevorzugt setzt man reinen Wasserstoff (Reinheit ≥ 99,9 Vol.-%, besonders ≥ 99,95 Vol.-%, insbesondere ≥ 99,99 Vol.-%) ein.

Aufgrund der hohen Katalysatoraktivität benötigt man vergleichsweise geringe Mengen an Katalysator bezogen auf das eingesetzte Edukt. So wird man bei der diskontinuierlichen Suspensionsfahrweise bevorzugt weniger als 5 mol-%, z.B. 0,2 Mol-% bis 2 Mol-% Aktivmetall, bezogen auf 1 Mol Edukt, einsetzen. Bei kontinuierlicher Ausgestaltung des Hydrierverfahrens wird man üblicherweise das zu hydrierende Edukt mit einer Belastung von 0,05 bis 3 kg/(l(Katalysator)•h), insbesondere 0,15 bis 2 kg/(l(Katalysator)•h), über den Katalysator führen.

### INSBESONDERS BEVORZUGTE HYDRIERVERFAHREN

Bei der Aromatenhydrierung umfassend einen Regenerierungsgehalt erfolgt im Allgemeinen bei einer Temperatur von 75°C bis 170°C, bevorzugt 80°C bis 160°C. Der Druck beträgt im Allgemeinen ≤ 50 bar, bevorzugt 10 bis 45 bar, besonders bevorzugt 15 bis 40 bar, ganz besonders bevorzugt 18 bis 38 bar.

Beispielsweise wird im Rahmen des vorliegenden Verfahrens Benzol bei einem Druck von etwa 20 bar zu Cyclohexan hydriert. Phthalate werden bei einem Druck von ≥ 10 bar zu den entsprechenden Cyclohexandicarbonsäurderivaten hydriert. Insbesondere wird Diisononylphthalat bei einem Druck im Bereich von etwa 200 bis etwa 250 bar zu Diisononylcyclohexandicarboxylat hydriert.

Die Hydrierung kann im Allgemeinen in der Suspensions- oder Festbettfahrweise durchgeführt werden, wobei eine Durchführung in der Festbettfahrweise bevorzugt ist. Besonders bevorzugt wird das Hydrierverfahren mit Flüssigkeitsumlauf durchgeführt, wobei die Hydrierwärme über einen Wärmetauscher abgeführt und genutzt werden kann. Das Zulauf/Kreislauf-Verhältnis beträgt bei einer Durchführung des Hydrierverfahrens mit Flüssigkeitsumlauf von im Allgemeinen 1:5 bis 1:40, bevorzugt von 1:10 bis 1:30.

Um einen vollständigen Umsatz zu erzielen, kann eine Nachreaktion des Hydrieraustrags erfolgen. Dazu kann der Hydrieraustrag im Anschluss an das Hydrierverfahren in der Gasphase oder in der Flüssigphase im geraden Durchgang, durch einen nachgeschalteten Reaktor geleitet werden. Der Reaktor kann bei Flüssigphasenhydrierung in Rieselfahrweise betrieben oder geflutet betrieben werden. Der Reaktor ist mit dem erfindungsgemäßen oder mit einem anderen, dem Fachmann bekannten, Katalysator befüllt.

### REGENERIERUNGSSCHRITT

Bei Hydrierverfahren, in denen die oben dargestellten Katalysatoren eingesetzt werden, ist nach einer gewissen Katalysatorstandzeit eine Desaktivierung zu beobachten. Ein solcher deaktivierter Rutheniumkatalysator kann durch Spülen in den Zustand der ursprünglichen Aktivität zurückgeführt werden. Die Aktivität lässt sich bis auf > 90 %, vorzugsweise > 95 %, mehr bevorzugt > 98 %, insbesondere > 99 %, meist bevorzugt > 99,5 % des Ursprungswertes wieder herstellen. Die Desaktivierung wird auf Spuren beziehungsweise Reste an dem Katalysator adsorbiertem Wasser zurückgeführt. Dies lässt sich überraschenderweise durch das Spülen mit Inertgas rückgängig machen. Das erfindungsgemäße Regenerierungsverfahren lässt sich somit auch als Trocknung des Katalysators oder Entfernen von Wasser von diesem bezeichnen.

"Spülen" bedeutet, dass der Katalysator mit Inertgas in Kontakt gebracht wird. Normalerweise wird dazu durch geeignete, dem Fachmann bekannte konstruktive Maßnahmen das Inertgas über den Katalysator geleitet.

Das Spülen mit Inertgas wird bei einer Temperatur von ungefähr 10 bis 350 °C, bevorzugt von ungefähr 50 bis 250 °C, besonders bevorzugt von ungefähr 70 bis 180 °C, am meisten bevorzugt von ungefähr 80 bis 130 °C durchgeführt.

Die beim Spülen angelegten Drücke betragen 0,5 bis 5 bar, vorzugsweise 0,8 bis 2 bar, insbesondere 0,9 bis 1,5 bar.

Erfindungsgemäß wird die Behandlung des Katalysators mit einem Inertgas durchgeführt. Bevorzugte Inertgase umfassen Stickstoff, Kohlendioxid, Helium, Argon, Neon, Krypton, Radon, Xenon und Mischungen daraus durchgeführt. Am meisten bevorzugt ist Stickstoff.

Gemäß der Erfindung wird das erfindungsgemäße Verfahren des Regenerierens ohne Ausbau des Katalysators in demselben Reaktor durchgeführt, in dem die Hydrierung stattgefunden hat. In besonders vorteilhafter Weise wird das Spülen des Katalysators gemäß der vorliegenden Erfindung bei Temperaturen und Drücken im Reaktor durchgeführt, die entsprechend oder ähnlich der Hydrierungsreaktion sind, wodurch eine nur sehr kurze Unterbrechung des Reaktionsprozesses resultiert.

Gemäß der vorliegenden Erfindung wird das Spülen mit Inertgas mit einem Volumenstrom von 20 bis 200 NI/h, bevorzugt mit einem Volumenstrom von 50 bis 200 NI/h pro Liter Katalysator durchgeführt.

Das Spülen mit Inertgas wird bevorzugt über eine Zeitdauer von 10 bis 50 Stunden, besonders bevorzugt von 10 bis 20 Stunden, durchgeführt. Beispielsweise beträgt die errechnete Trockenzeit des Katalysatorbettes einer großtechnischen Cyclohexan-Produktionsanlage mit einer angenommenen Feuchte von 2 beziehungsweise 5 Gew.-% näherungsweise 18 beziehungsweise 30 Stunden. Das Spülen kann in dem erfindungsgemäßen Verfahren sowohl in abwärts gerichteter Richtung (down-flow) als auch in aufwärts gerichteter Richtung (up-flow) durchgeführt werden.

Gegenstand der vorliegenden Erfindung ist ein integriertes Verfahren zum Hydrieren eines aromatischen Kohlenwasserstoffs in Gegenwart eines Rutheniumkatalysators gemäss Anspruch 1.

Der erfindungsgemäße eingesetzte Wasserstoff enthält vorzugsweise keine schädlichen Katalysatorgifte, wie beispielsweise CO. Beispielsweise können Reformergase verwendet werden. Vorzugsweise wird reiner Wasserstoff als Hydriergas verwendet.

Das Verfahren eignet sich weiterhin zur Trocknung von Katalysatoren, die während verschiedener Vorgänge wie Wartung oder Lagerung Wasser aufgenommen haben.

Die Erfindung soll anhand der folgenden Beispiele näher erläutert werden:

### Beispiele

### Herstellungsbeispiel des Rutheniumkatalysators

Ein meso-/makroporöser Aluminiumoxidträger in Form von 3 bis 5 mm-Kugeln mit einem Gesamtvolumen von 0,44 cm³/g, wobei 0,09 cm³/g (20% des Gesamtporenvolumens) von Poren mit einem Durchmesser im Bereich von 50 nm bis 10.000 nm und 0,35 cm³/g (80% des Gesamtporenvolumens) von Poren mit einem Durchmesser im Bereich von 2 nm bis 50 nm gebildet werden, einem mittleren Porendurchmesser im Bereich von 11 nm und einer Oberfläche von 286 m²/g wurde mit einer wässrigen Ruthenium-(III)-nitrat-Lösung getränkt. Das während des Tränkens aufgenommene Lösungsvolumen entsprach dabei in etwa dem Porenvolumen des verwendeten Trägers. Anschließend wurde der mir der Ruthenium-(III)-nitrat-Lösung getränkte Träger bei 120°C getrocknet und bei 200°C im Wasserstoffstrom aktiviert (reduziert). Der so hergestellte Katalysator enthielt 0,5 Gew.-% Ruthenium, bezogen auf das Gewicht des Katalysators. Die Rutheniumoberfläche betrug 0,72 m²/g, das Verhältnis von Ruthenium- zu Trägeroberfläche lag bei 0,0027.

### Beispiel 1 Sorptionsuntersuchungen

Durch Sorptionsmessungen von Wasserdampf an dem wie vorstehend beschrieben hergestellten Katalysator (0,5 % Ru/γ-Al₂O₃), wurde die Affinität des Katalysators zum Wasser ermittelt.

Es zeigte sich, dass der Katalysator schon bei geringen relativen Dampfdrücken von 30% eine Wassermenge von 5% sorbiert. Falls im Reaktor beziehungsweise in den Einsatzstoffen Wasser auch nur in Spuren vorhanden ist, kann dieses Wasser am Katalysator sorbiert werden.

### Beispiel 2 Standzeitversuch bei der Hydrierung von Benzol

In einer Anlage zur Herstellung von Cyclohexan unter Verwendung eines Ruthenium/Aluminiumoxidkatalysators mit 0,5 % Ru auf einem Träger aus γ-Al₂O₃ wird im Produktstrom ein stetiges Nachlassen der Katalysatoraktivität und ein zunehmender Benzolgehalt beobachtet. Ein weiteres Verfolgen der Reaktion zeigt, dass während eines Katalysatorstandzeittests bei der Hydrierung von Benzol der Restbenzolgehalt nach dem Hauptreaktor innerhalb einer Laufzeit von ca. 3.400 h von wenigen Hundert ppm auf einige Tausend ppm ansteigt. Eine Berechnung ergibt, dass bei der Zuführung von 16.620 kg/h Benzol mit einem Wassergehalt von 30 bis 50 ppm, 0,8 kg Wasser pro Stunde in die Anlage eingebracht werden. Hinzu kommen noch weitere 3,5 kg/h Wasser, welche aus dem Wasserstoffgas stammen.

Bei Abschalten der Anlage bei 3.394 Betriebsstunden lief die Anlage mit einem Restbenzolgehalt von 0,2 % bei einer Belastung von 0,6 g_{Benzol/}ml_{Kat}·h. Während des Abschaltens wurde die Anlage bei einer Temperatur von 70 bis 100°C mit Stickstoff ausgepresst und dann entspannt. Nach dem Anfahren lieferte die Anlage einen Restbenzolgehalt von 0,01 % bis 0,04 % bei einer Belastung von 0,6 9_{Benzol}/Ml_{Kat}·h.

Dieser beobachtete Effekt des Trocknens des Katalysators wurde nach 7.288 Betriebsstunden erneut verifiziert. Bei einer Belastung von 0,9 g_{Benzol}/Ml_{Kat}·h lag der Restbenzolgehalt am Ende der Anlage bei 0,2% und stieg sogar auf 0,56% an. Nach dem Abstellen der Anlage wurde der Katalysator über einen Zeitraum von 34 h bei 110°C mit 100 Nl/h Stickstoff getrocknet. Nach dem Anlaufen der Anlage mit einer Belastung von 0,6 g_{Benzol}/ml_{Kat}·h lag der Restbenzolgehalt bei 0,03 % bis 0,07 %, was auf eine deutliche Steigerung der Katalysatoraktivität durch das Trocknen zurückgeführt werden kann.

In beiden Fällen führte die Trocknung des Katalysators zu einer signifikant höheren Katalysatoraktivität, die nahe oder gleich der ursprünglichen Katalysatoraktivität liegt.

### Beispiel 3 Untersuchung des Wassereinflusses auf die Benzolhydrierung

Zur Simulierung des Wassereinflusses auf die Hydrierung von Benzol mit einem Rutheniumkatalysator wurden Versuchsreihen in Autoklavenversuchen vor und nach der Sättigung des Katalysators mit Wasser sowie nach Trocknung des Katalysators durchgeführt. Im Druckbehälter wurden eine 5 %ige Lösung von Benzol in Cyclohexan mit dem Rutheniumkatalysator vorgelegt, auf die Reaktionstemperatur von 100 °C aufgeheizt und der Reaktionsverlauf bei 32 bar Wasserstoffdruck durch regelmäßige Probennahme verfolgt. Die Proben wurden im Anschluss durch Gaschromatographie untersucht.

Es wurden 23 Hydrierversuche durchgeführt, der Katalysator im Anschluss ins Wasser gestellt. Danach wurden 13 weitere Hydrierversuche durchgeführt. Der Katalysator zeigte eine deutlich geringere, aber nahezu konstante Aktivität. Nach Trocknung des Katalysators im Stickstoffstrom bei 100°C in einem Reaktionsrohr wurden 5 weitere Versuche durchgeführt; der Katalysator zeigte eine ähnliche Hydrieraktivität wie vor der Sättigung mit Wasser.

Die Versuche belegen, dass die Aktivität des eingesetzten Ruthenium/AluminiumoxidKatalysators nach Kontakt mit Wasser signifikant nachlässt, der Katalysator aber durch Trocknung im Stickstoffstrom wieder reaktiviert und die Anfangsaktivität nahezu vollständig wieder hergestellt werden kann.

## Patentansprüche

1. Integriertes Verfahren zur Hydrierung von hydrierbare Gruppen enthaltenden Substanzen umfassend die folgenden Schritte:
a) Bereitstellen einer zu hydrierenden Substanz und eines Rutheniumkatalysators,
b) Hydrieren der Substanz in der Flüssigphase oder in der Gasphase in Abwesenheit oder Gegenwart eines Lösungs- oder Verdünnungsmittels durch Kontakt mit Wasserstoff in Gegenwart des Rutheniumkatalysators, bis der Katalysator eine reduzierte Hydrieraktivität aufweist,
c) Regenerierung des Katalysators, bestehend aus dem Spülen mit Inertgas, wobei Wasser von dem Katalysator entfernt wird,
bis zum Erreichen der ursprünglichen Aktivität oder eines Teils der ursprünglichen Aktivität, und
d) gegebenenfalls Wiederholung der Schritte a) bis c), wobei Schritt c) ohne Ausbau des Katalysators durchgeführt wird.

2. Verfahren nach Anspruch 1, **dadurch gekennzeichnet, dass** das Spülen mit Inertgas bei einer Temperatur von 10 bis 350 C° durchgeführt wird.

3. Verfahren nach Anspruch 1 oder 2, **dadurch gekennzeichnet, dass** das Spülen bei einem Druck von 0,5 bis 5 bar durchgeführt wird.

4. Verfahren nach einem der Ansprüche 1 bis 3, **dadurch gekennzeichnet, dass** das Inertgas ausgewählt ist aus Stickstoff, Kohlendioxid, Helium, Argon, Neon und Mischungen daraus.

5. Verfahren nach einem der Ansprüche 1 bis 4, **dadurch gekennzeichnet, dass** das Spülen mit Inertgas mit einem Volumenstrom von 20 bis 200 Nl/h, pro Liter Katalysator durchgeführt wird.

6. Verfahren nach einem der Ansprüche 1 bis 5, **dadurch gekennzeichnet, dass** Spülen mit Inertgas über einen Zeitraum von 10 bis 50 Stunden durchgeführt wird.

7. Verfahren nach einem der Ansprüche 1 bis 6, **dadurch gekennzeichnet, dass** der Regenerierungsschritt durchgeführt wird, bis eine Aktivität von > 90 % des Ursprungswertes erreicht ist.

8. Verfahren nach einem der Ansprüche 1 bis 7, **dadurch gekennzeichnet, dass** der Rutheniumkatalysator zur Hydrierung von Aromaten geeignet ist.

9. Verfahren nach einem der Ansprüche 1 bis 8, **dadurch gekennzeichnet, dass** der Rutheniumkatalysator ausgewählt ist aus den nachfolgenden Gruppen:
a) Katalysator enthaltend als Aktivmetall Ruthenium alleine oder zusammen mit mindestens einem Metall der I., VII. oder VIII. Nebengruppe des Periodensystems in einer Menge von 0,01 bis 30 Gew.-%, bezogen auf das Gesamtgewicht des Katalysators, aufgebracht auf einem Träger, und wobei 10 bis 50 % des Porenvolumens des Trägers von Makroporen mit einem Porendurchmesser im Bereich von 50 nm bis 10.000 nm und 50 bis 90 % des Porenvolumens des Trägers von Mesoporen mit einem Porendurchmesser im Bereich von 2 bis 50 nm gebildet werden, wobei sich die Summe der Porenvolumina zu 100 % addiert, und
b) Schalenkatalysator enthaltend als Aktivmetall Ruthenium alleine oder zusammen mit mindestens einem weiteren Metall der Nebengruppen IB, VIIB oder VIII des Periodensystems der Elemente (CAS-Version), aufgebracht auf einen Träger enthaltend Siliziumdioxid als Trägermaterial, **dadurch gekennzeichnet, dass** die Menge des Aktivmetalls < 1 Gew.-% beträgt, bezogen auf das Gesamtgewicht des Katalysators, und mindestens 60 Gew.-% des Aktivmetalls in der Schale des Katalysators bis zu einer Eindringtiefe von 200 µm vorliegen, ermittelt mittels SEM-EPMA (EDXS)

10. Verfahren nach Anspruch 9, **dadurch gekennzeichnet, dass** es sich bei dem Katalysator um Katalysator a) handelt und das mindestens eine Metall der I., VII., oder VIII. Nebengruppe des Periodensystems Platin, Kupfer, Rhenium, Kobalt, Nickel oder ein Gemisch aus zwei oder mehr davon ist.

11. Verfahren nach Anspruch 9 , **dadurch gekennzeichnet, dass** es sich bei dem Katalysator um Katalysator b) handelt und das mindestens eine Metall der I., VII., oder VIII. Nebengruppe des Periodensystems Platin, Rhodium, Palladium, Iridium, Kobalt, Nickel oder ein Gemisch aus zwei oder mehr davon oder Kupfer und/oder Rhenium ist.

12. Verfahren nach Anspruch 10, **dadurch gekennzeichnet, dass** es sich bei dem Katalysator um Katalysator a) handelt und der Träger Aktivkohle, Siliziumcarbid, Aluminiumoxid, Titanoxid, Zirkoniumoxid, Magnesiumoxid, Zinkoxid oder ein Gemisch aus zwei oder mehr davon ist.

13. Verfahren nach Anspruch 11, **dadurch gekennzeichnet, dass** es sich bei dem Katalysator um Katalysator b) handelt und das Trägermaterial zu wenigstens 90 Gew.-% aus amorphem Siliziumdioxid besteht, wobei die verbleibenden 10 Gew.-% des Trägermaterials auch ein anderes oxidisches Material sein können.

14. Verfahren nach einem der Ansprüche 1 bis 13, **dadurch gekennzeichnet, dass** Benzol zu Cyclohexan beziehungsweise Diisononylphthalat zu Diisononylcyclohexan-dicarboxylat umgesetzt wird.

15. Verfahren nach einem der Ansprüche 1 bis 14, **dadurch gekennzeichnet, dass** die zu hydrierende Substanz ein Aromat ist.

## Claims

1. An integrated process for the hydrogenation of substances comprsing hydrogenatable groups comprising the following steps:
a) provision of a substance to be hydrogenated and a ruthenium catalyst;
b) hydrogenation of the substance in the liquid phase or in the gas phase in the absence or presence of a solvent or diluent by contact with hydrogen in the presence of the ruthenium catalyst until the catalyst has a reduced hydrogenation activity,
c) regeneration of the catalyst, consisting of flushing with inert gas, with water being removed from the catalyst,
until the original activity or part of the original activity has been attained, and
d) optionally, repetition of the steps a) to c), with step c) being carried out without removal of the catalyst.

2. The process according to claim 1, wherein the flushing with inert gas is carried out at a temperature of from 10 to 350°C.

3. The process according to claim 1 or 2, wherein the pressure applied during flushing is from 0.5 to 5 bar.

4. The process according to any of claims 1 to 3, wherein the inert gas is selected from among nitrogen, carbon dioxide, helium, argon, neon and mixtures thereof.

5. The process according to any of claims 1 to 4, wherein the flushing with inert gas is carried out at a volume flow of from 20 to 200 standard 1/h per liter of catalyst.

6. The process according to any of claims 1 to 5, wherein flushing with inert gas is carried out for a time of from 10 to 50 hours.

7. The process according to any of claims 1 to 6, wherein the regeneration step is carried out until an activity of > 90% of the original value is attained.

8. The process according to any of claims 1 to 7, wherein the ruthenium catalyst is suitable for the hydrogenation of aromatics.

9. The process according to any of claims 1 to 8, wherein the ruthenium catalyst is selected from among the following groups:
a) catalyst comprising, as active metal, either ruthenium alone or ruthenium together with at least one metal of transition group I, VII or VIII of the Periodic Table in an amount of from 0.01 to 30% by weight, based on the total weight of the catalyst, applied to a support, wherein from 10 to 50% of the pore volume of the support is formed by macropores having a pore diameter in the range from 50 nm to 10 000 nm and from 50 to 90% of the pore volume of the support being formed by mesopores having a pore diameter in the range from 2 to 50 nm, with the sum of the pore volumes being 100%, and
b) coated catalyst comprising, as active metal, either ruthenium alone or ruthenium together with at least one further metal of transition group IB, VIIB or VIII of the Periodic Table of the Elements (CAS version) applied to a support comprising silicon dioxide as support material, wherein the amount of active metal is < 1% by weight, based on the total weight of the catalyst, and at least 60% by weight of the active metal is present in the shell of the catalyst to a penetration depth of 200 µm, determined by means of SEM-EPMA (EDXS).

10. The process according to claim 9, wherein the catalyst is catalyst a) and the at least one metal of transition group I, VII or VIII of the Periodic Table is platinum, copper, rhenium, cobalt, nickel or a mixture of two or more thereof.

11. The process according to claim 9, wherein the catalyst is catalyst b) and the at least one metal of transition group I, VII or VIII of the Periodic Table is platinum, rhodium, palladium, iridium, cobalt, nickel or a mixture of two or more thereof, or copper and/or rhenium.

12. The process according to claim 10, wherein the catalyst is catalyst a) and the support is activated carbon, silicon carbide, aluminum oxide, titanium oxide, zirconium oxide, magnesium oxide, zinc oxide or a mixture of two or more thereof.

13. The process according to claim 11, wherein the catalyst is catalyst b) and the support material comprises at least 90% by weight of amorphous silicon dioxide, with the remaining 10% by weight of the support material also being able to be another oxidic material.

14. The process according to any of claims 1 to 13, wherein benzene is converted into cyclohexane or diisononyl phthalate is converted into diisononylcyclohexanecarboxylate.

15. The process according to any of claims 1 to 14, wherein the substance to be hydrogenated is an aromatic.

## Revendications

1. Procédé intégré d'hydrogénation de substances contenant des groupes hydrogénables, comprenant les étapes suivantes :
a) la préparation d'une substance à hydrogéner et d'un catalyseur à base de ruthénium,
b) l'hydrogénation de la substance dans la phase liquide ou dans la phase gazeuse en l'absence ou en la présence d'un solvant ou d'un diluant par mise en contact avec de l'hydrogène en présence du catalyseur à base de ruthénium, jusqu'à ce que le catalyseur présente une activité d'hydrogénation réduite,
c) la régénération du catalyseur, constituée par un rinçage avec un gaz inerte, l'eau étant éliminée du catalyseur,
jusqu'à atteindre l'activité initiale ou une partie de l'activité initiale, et
d) éventuellement la répétition des étapes a) à c), l'étape c) étant réalisée sans démontage du catalyseur.

2. Procédé selon la revendication 1, **caractérisé en ce que** le rinçage avec un gaz inerte est réalisé à une température de 10 à 350 °C.

3. Procédé selon la revendication 1 ou 2, **caractérisé en ce que** le rinçage est réalisé à une pression de 0,5 à 5 bar.

4. Procédé selon l'une quelconque des revendications 1 à 3, **caractérisé en ce que** le gaz inerte est choisi parmi l'azote, le dioxyde de carbone, l'hélium, l'argon, le néon et leurs mélanges.

5. Procédé selon l'une quelconque des revendications 1 à 4, **caractérisé en ce que** le rinçage avec un gaz inerte est réalisé avec un débit volumique de 20 à 200 Nl/h, par litre de catalyseur.

6. Procédé selon l'une quelconque des revendications 1 à 5, **caractérisé en ce que** le rinçage avec un gaz inerte est réalisé pendant une durée de 10 à 50 heures.

7. Procédé selon l'une quelconque des revendications 1 à 6, **caractérisé en ce que** l'étape de régénération est réalisée jusqu'à ce qu'une activité > 90 % de la valeur initiale soit atteinte.

8. Procédé selon l'une quelconque des revendications 1 à 7, **caractérisé en ce que** le catalyseur à base de ruthénium est approprié pour l'hydrogénation de composés aromatiques.

9. Procédé selon l'une quelconque des revendications 1 à 8, **caractérisé en ce que** le catalyseur à base de ruthénium est choisi dans les groupes suivants :
a) un catalyseur contenant en tant que métal actif du ruthénium seul ou conjointement avec au moins un métal du groupe de transition I, VII ou VIII du tableau périodique en une quantité de 0,01 à 30 % en poids, par rapport au poids total du catalyseur, appliqué sur un support, 10 à 50 % du volume poreux du support étant formé par des macropores ayant un diamètre de pore dans la plage allant de 50 nm à 10 000 nm, et 50 à 90 % du volume poreux du support étant formé par des mésopores ayant un diamètre de pore dans la plage allant de 2 à 50 nm, la somme des volumes poreux étant de 100 %, et
b) un catalyseur à enveloppe contenant en tant que métal actif du ruthénium seul ou conjointement avec au moins un autre métal des groupes de transition IB, VIIB ou VIII du tableau périodique des éléments (version CAS), appliqué sur un support contenant du dioxyde de silicium en tant que matériau support, **caractérisé en ce que** la quantité du métal actif est < 1 % en poids, par rapport au poids total du catalyseur, et au moins 60 % en poids du métal actif se trouve dans l'enveloppe du catalyseur jusqu'à une profondeur de pénétration de 200 µm, déterminée par SEM-EPMA (EDXS).

10. Procédé selon la revendication 9, **caractérisé en ce que** le catalyseur est le catalyseur a) et ledit au moins un métal du groupe de transition I, VII ou VIII du tableau périodique est le platine, le cuivre, le rhénium, le cobalt, le nickel ou un mélange de deux ou plus d'entre eux.

11. Procédé selon la revendication 9, **caractérisé en ce que** le catalyseur est le catalyseur b) et ledit au moins un métal du groupe de transition I, VII ou VIII du tableau périodique est le platine, le rhodium, le palladium, l'iridium, le cobalt, le nickel ou un mélange de deux ou plus d'entre eux, ou le cuivre et/ou le rhénium.

12. Procédé selon la revendication 10, **caractérisé en ce que** le catalyseur est le catalyseur a), et le support est le charbon actif, le carbure de silicium, l'oxyde d'aluminium, l'oxyde de titane, l'oxyde de zirconium, l'oxyde de magnésium, l'oxyde de zinc ou un mélange de deux ou plus d'entre eux.

13. Procédé selon la revendication 11, **caractérisé en ce que** le catalyseur est le catalyseur b), et le matériau support est constitué par au moins 90 % en poids de dioxyde de silicium amorphe, les 10 % en poids restants du matériau support pouvant également être un autre matériau oxydique.

14. Procédé selon l'une quelconque des revendications 1 à 13, **caractérisé en ce que** du benzène est transformé en cyclohexane ou du phtalate de diisononyle en dicarboxylate de diisononylcyclohexane.

15. Procédé selon l'une quelconque des revendications 1 à 14, **caractérisé en ce que** la substance à hydrogéner est un composé aromatique.
